# EUROPEAN PATENT APPLICATION

(11) **EP 2 345 421 A1**
(43) Date of publication of application: **20.07.2011**
(21) Application number: 11160025.0
(22) Date of filing: 22.06.2004
(51) Int. Cl.: A61K 39/35, A61K 38/01

(54) **Epitope composition for sublingual or enteric administration prepared by hydrolysis of antigenic structures with chymotrypsin**

(30) Priority: 19.12.2003 US 530629 P; 23.06.2003 EP 03014020; 19.12.2003 EP 03029356
(62) Divisional of application: 04740162.5
(71) Applicant: Biotech Tools S.A., 1120 Brussels (BE)
(72) Inventor: Henot, Frédéric, 1040 Bruxelles (BE); Legon, Thierry, 3360 Korbeek Lo (BE); Duchateau, Jean, 7060 Soignies (BE)
(74) Representative: von Kreisler Selting Werner

(57) **Abstract**

A pharmaceutical composition for sublingual or buccal administration comprising at least one substance obtainable by hydrolysis with chymotrypsin of an antigenic structure which induces graft rejection, allergic reaction or autoimmune disease.

## Description

The present invention relates to a pharmaceutical composition and the use of the pharmaceutical composition.

### Background of the invention

There are a large number of severe diseases based on unwanted recognition of antigens by antibodies or are mediated by T-cells. These diseases include allergic reactions and autoimmune diseases and antigen/antibody reactions are also responsible for graft rejections after transplantation.

Beside a large number of medicaments for suppression of the immune reaction or the symptoms of the diseases no satisfying causal therapy is available. Despite a large number of experiments and studies, there is still a need for new pharmaceutical compositions.

WO 88/10120 discloses a method of treating a T-cell mediated autoimmune disease in animals by oral or enteral administration of autoantigens, fragments of autoantigens or analogs structurally related to those autoantigens, which are specific for the particular autoimmune disease.

US 6,312,711 discloses a pharmaceutical and/or food composition comprising at least one of the conformational or sequential epitopes of an antigenic structure related to graft rejection, allergic reaction or autoimmune reaction together with stress protein selected from the group of stress protein GroEL, GrpE, DnaK and DnaJ.

Pecquet et al., in Vaccine 18 (2000) 1196 to 1202, disclose the induction of oral tolerance in mice by entrapped ß-lactoglobulin. As discussed in this article, controversial results have been obtained by different groups in connection with similar studies.

### Aim of the invention

The aim of the present invention is to provide a novel pharmaceutical composition designed to modify the immune response of patients towards diseases associated with an allergic or autoimmune reaction or towards graft rejection.

A further aim was to provide a composition which produces reliable and reproducible results.

Another aim is to provide a method for treatment or prevention of graft rejection, allergic reaction or autoimmune disease.

### Summary of the invention

In one embodiment of the invention, the invention provides a pharmaceutical composition for sublingual, buccal or enteric administration comprising at least one substance obtainable by hydrolysis with chymotrypsin of an antigenic structure which induces graft rejection, allergic reaction or autoimmune disease.

Another embodiment of the invention is the use of the composition of the invention for the treatment or prevention of graft rejection, allergic reaction or autoimmune disease or for elicting oral tolerance and/or the induction of cells that may produce immunosuppressive cytokines, more preferably TGF-beta and/or IL-4 and/or IL-10.

The compositions of the present invention are especially useful to treat or prevent graft rejection, allergic reaction or autoimmune disease in mammals, especially humans.

In a further embodiment, the invention provides a process for the preparation of the pharmaceutical composition of the invention comprising the steps of
- hydrolyzing with chymotrypsin or proteins having a chymotrypsin-like activity an antigenic structure which induces graft rejection, allergic reaction or autoimmune disease to obtain at least one substance
- formulating the at least one substance for enteric, sublingual or buccal administration.

### Detailed description of the invention

The present invention provides a pharmaceutical composition for sublingual, buccal or enteric administration comprising at least one substance obtainable by hydrolysis with chymotrypsin of an antigenic structure which induces graft rejection, allergic reaction or autoimmune disease.

Graft rejection, allergic reaction or autoimmune diseases are hypersensitivity reactions of immediate or delayed type brought about by contact in particular with an allergen (this reaction can be immediate and specific (anaphylaxis, urticarier, etc.) or delayed over time) or autoimmune diseases and disorders of the immune system of immediate or delayed type associated with graft rejections of host against graft type and a graft against host type.

Autoimmune diseases or disorders are a state of immunization of an individual against his or her own constituents and the phenomenon of graft rejection is a state of immunization of an individual against foreign constituents brought into contact with the patients. Typical autoimmune diseases are inter alias Systemic Lupus erytematosus disease, Sjögren's disease, rheumatoid polyarthritis, as well as pathologies such as sarcoidosis and osteopenia, spondylarthritis, scleroderma, multiple sclerosis, amyotrophic lateral sclerosis, hyperthyroidism, Addison's disease, autoimmune hemolytic anemia, Crohn's disease, Goddpasture's syndrome, Graves' disease, Hashimoto's thyroiditis, idiopathic purpural hemorrhage, insulein-dependent diabetes, myasthenia, pemphigus vulgaris, pernicious anemia, poststreptococcal glomerulonephrtitis, psoriasis and spontaneous sterility.

The term "antigenic structure" covers macromolecules such as allergens made of peptides, lipids, polysaccharides and/or nucleic acids. Typical antigenic structures are inter alias insulin, thyroglobulin, thyroid peroxidase, type II collagen, gliadin, GAD65, proteolipid protein, S-antigen, acetylcholin receptor, haptenized colonic proteins, interphotoreceptor retinoid binding protein, myelin basic protein, myelin oligodendrocyte glycoprotein, peripheral nerve P2, cytoplasmic TSH receptor, intrinsic factor, lens proteins, platelets, nucleoproteins such as histones, heat shock proteins, MHC I, MHC II, MHC-peptides complexes, milk allergens, venom allergens, egg allergens, weed allergens, grass allergens, tree allergens, shrub allergens, flower allergens, grain allergens, fungi allergens, fruit allergens, berry allergens, nut allergens, seed allergens, bean allergens fish allergens, shellfish allergens, meat allergens, spices allergens, insect allergens, mite allergens, animal allergens, animal dander allergens, allergens of Hevea brasiliensis, coagulation factors and blood group antigens.

According to the invention, the composition comprises at least one substance which is obtainable by hydrolysis of an antigenic structure, that is according to the invention not complete antigenic structure are used in the pharmaceutical composition but fragments thereof.

Surprisingly, hydrolysis with chymotrypsin provides improved pharmaceutical compositions compared to hydrolysis with pepsin or other trypsin. However, without to be bound to this observation, the hydrolysis can also be performed with any other protease selected from the list according to the nomenclature Committee of the international union of biochemistry and molecular biology, the list of MEROPS database http://www.merops.co.uk and Nucleid Acids Res. 2004:32 Database issue:D160-4., and of Barret AJ, Rawlings ND Woessner JF (eds) 1998 Handbook of Proteolytic Enzymes, Academic Press Londong.

Such substances can either be prepared by hydrolysis but they can also be prepared by synthetic methods.

In case of an hydrolysis, the antigenic structure can be modified prior to hydrolysis either by physical e.g. heating, high mechanical pressure or by chemical methods e.g. reductive reagents (such as thioredoxin activated either by NADPH via NADP-thioredoxin-reductase or by dithiothreitol) oxidative reagents, alkylating reagents, urea, guanidinium chloride. The antigenic structure can also be treated prior to hydrolysis with enzymes, such as but not limited to lipases, protein kinases, protein phosphatases, and N-glycosylases.

What is important according to the invention is that the pharmaceutical composition is prepared for sublingual, enteric or buccal administration.

"Sublingual administration" and "buccal administration" are methods wherein the substance is combined in a pharmaceutical formulation which allows absorption of the at least one substance in the mouth mucosa. Sublingual administration involves the patients holding a (sublingual) pharmaceutical composition or dosage from under their tongue while the substance diffuses into the mouth, through the mucosa lining the mouth. In buccal administration, the patients hold the (buckle) pharmaceutical composition or dosage from between their cheek and gingiva (gum) instead of under the tongue. The buccal administration can be chewed to allow faster buccal absorption or release; the present invention therefore provides in a preferred embodiment a gum-based formulation or a chewing gum formulation.

"Enteric administration" is a method wherein the substance is in a pharmaceutical formulation which protects the active ingredient from absorption and/or degradation prior to entry into the intestine. Preferably absorption is effected in the ileum, duodenum or jejunum. In one preferred embodiment, the said pharmaceutical formulation can be a suppository.

Especially suitable formulation includes coating with polymers, e.g. as sold under the trademark Eudragit^{®}, commercially available from Degussa, Germany. Eudragid^{®} polymers are suitable for solid oral formulations which are released in the intestine. In a preferred embodiment, suitable pharmaceutical formulations are comprising any needed binders or excipients for the neutralization of hydrochloric acid (gastric acid secretion) and/or the inhibition of pepsin and/or the stimulation of bicarbonate and mucus secretion in a patient.

Neutralization of hydrochloric acid and/or inhibition of pepsin in the stomach can be achieved for example with sucralfate or a proton-binding polymer, such as but not limited to polyethylenimine, or any neutralizing anti-acid (antacid) or any acid blocker selected from the group consisting of aluminum salts, bismuth salts, magnesium salts, sodium bicarbonate, potassium bicarbonate, potassium citrate, sodium potassium tartrate, tricalcium phosphate, and mixtures thereof.

Some other types of acid blockers that can be used in the suitable formulation are called gastric proton pump inhibitors (or gastric H+/K+ ATPase inhibitors), prostaglandin analogues and histamine H2-receptor antagonists. These include, but are not limited to, misoprostol, ranitidine (used in ZANTAC®), cimetidine (used in TAGAMET®), nizatidine (used in AXID®), famotindine (used in PEPCID®), sufotidine, roxatidine, bisfentidine, tiotidine, lamtidine, niperotidine, mifentidine, zaltindine, loxtidine, omeprazole (used in PRISOLEC®), and rabeprazole.

In another preferred embodiment, the suitable formulation comprises a microsphere of the said at least on substance bound to or encapsulated in an inert particle in whatever shape or form, having a mesh size of about 30 - 35 mesh (about 600 µm to 500 µm) or greater than about 40 mesh, and most preferably in the range of about 45 to 200 mesh, and may be for example a nonpareil, a silica powder, a salt crystal or a sugar crystal.

Without wishing to be bound to a theory, it is believed that former formulations of such antigenic structures were partially destroyed by the gastric juice. While this might have produced hydrolyzed fragments of the respective antigens, the amount of hydrolyzed peptides absorbed was highly dependent of the digestive activity of the patient and, therefore, it was highly variable.

Only with a pharmaceutical composition of the present invention, the composition can be produced with constant quality. By either sublingual, buccal or enteric administration, the amount of absorbed active ingredient can be tightly controlled.

It is important to identify the adequate amount for treatment or prevention of a respective diseases or disorders. Typical preferred amounts are in the range of 0.001 µg to 1000 µg per dosage unit and it is preferred that the dosage unit is 0.01 µg or more. In a more preferred embodiment, the dosage unit is 0.1 µg or more and in a very preferred embodiment, it is 1 µg or more.

It is also important that the amount of active ingredient is not too high. It is preferred that the amount of the at least one substance is 100 µg or less, 50 µg or less and more preferred 10 µg or less.

In one embodiment, these dosage units are calculated on the basis of a normal patient with a weight of 75 kg. Typically, 1 to 10 dosage units should be applied daily.

In one preferred embodiment the at least one substance (which is the active ingredient of the pharmaceutical composition of the present invention) is obtainable by hydrolysis of a protein. In a very preferred embodiment, the at least one substances is a peptide. The molecular weight of the peptide is preferably less than 30 kDa, more preferably less than 10 kDa. The at least one substance can be obtained by hydrolysis.

In another embodiment, the at least one substance obtainable by hydrolysis, preferably a peptide, can be further treated with proteins, such as but not limited to lipases, protein kinases, protein phosphatases, and N-glycosylases or with at least one chemical agent like, but not limited to hydroxylamine, cyanogen bromide.

In a preferred embodiment, the at least one substance could bind to specific immunoglobulins in the serum of a patient suffering from an allergy, an autoimmune disease or a graft rejection. Preferably, the immunoglobulins are IgG.

In another preferred embodiment, the at least one substance does not bind to specific immunoglobulins in the serum of patients suffering from an allergy, an autoimmune disease or a graft rejection.

Moreover, in a preferred embodiment, the at least one substance can bind to a heat shock protein (HSP).

In a further embodiment, the composition can comprise one or more "enhancers". Suitable enhancers are nucleoside triphosphates, nucleoside diphosphates, nucleoside monophosphates, nucleic acids, peptide nucleic acids, nucleosides or analogs thereof, immunosuppressive cytokines, 1,25-dihydroxyvitamin D3 or analogs thereof, lipopolysaccharides, endotoxins heat shock proteins, thioredoxin with either NADPH and NADP-Thioredoxin reductase or dithiothreitol, adrenergic receptor agonists such as salbutanol, adrenergic receptor antagonists such as butoxamine, compounds that regulate the expression of the adhesion molecule ICAM-1, N-acetyl-L-cysteine, γ-L-glutamyl-L-cysteinyl-glycine (reduced L-glutathione), alpha-2-macroglobulins, inducers for Foxp3 gene expression, flavonoids, isoflavonoids, pterocarpanoids, stilbenes such as resveratrol, tachykinin receptor antagonists, chymase inhibitors, a muco-adhesive agent for attaching the particle to the intestinal mucosal lining such as a plant lectin, zinc and zinc salts.

Other enhancers are polysaccharides, vitamines and compounds inducing expression of immunoproteasomes. A further preferred enhancer is a bacterial lysate, e.g. as described in EP 0 269 928 A2, GB 2240922 A or GB 2054374.

It is preferred that the pharmaceutical composition is free of heat shock proteins.

The composition of the present invention is especially useful for the treatment or prevention of graft rejection, allergic reaction or autoimmune disease. They are further suitable for elicting oral tolerance and/or the induction of cells that may produce immunosuppressive cytokines, more preferably TGF-beta and/or IL-4 and/or IL-10. In another embodiment, the induction of said cells with the compositions of the present invention is performed in-vitro and then the cells are "returned" in the body of a mammal, preferably a human, by for example intravenous introduction, surgical implantation or injection.

In a further embodiment the invention provides a process for the preparation of the composition which comprises the steps of
- hydrolyzing an antigenic structure which induces graft rejection, allergic reaction or autoimmune disease to obtain at least one substance
- formulating the at least one substance for enteric, sublingual or buccal administration.

As explained above, hydrolysis can be an enzymatic hydrolysis and hydrolysis with chymotrypsin is especially preferred. The invention is explained in more details by the following examples.

It will be appreciated that the pharmaceutical compositions and methods disclosed herein can be used prophylactically and therapeutically in a wide array of conditions. Thus, the embodiments of the present invention shown and described in the specification are only preferred embodiments and are not limiting in any way. Various changes, modifications or alterations to these embodiments may be made or resorted to without departing from the spirit of the invention and the scope of the claims.

### Examples

### Example 1

Four groups of mice were sensibilized against ß-lactoglobulin (BLG) according to the following protocol.

### Chymotrypsin digestion

One milligram of BLG is dissolved in 1 mL of Tris.HCL 40 mM, 10 mM CaCl₂ pH 8.0 and 20 µL of chymotrypsin solution (final ratio (w/w) protein/protease of 100: 1) is added to the protein. The resulting solution is incubated at 37°C for six hours. The solution is then centrifuged through a centricon YM-10 assembly to remove the remaining protein and chymotrypsin.

### HPLC analysis

The low molecular weight fractions are fractionated by reverse phase high pressure liquid chromatography (HPLC) using a Vydac C18 reverse phase column (HP32, 201TP52 C18, 250/2.1 mm, 5 µm). The elution of the peptides can be monitored at both OD 214 nm and OD 280 nm.

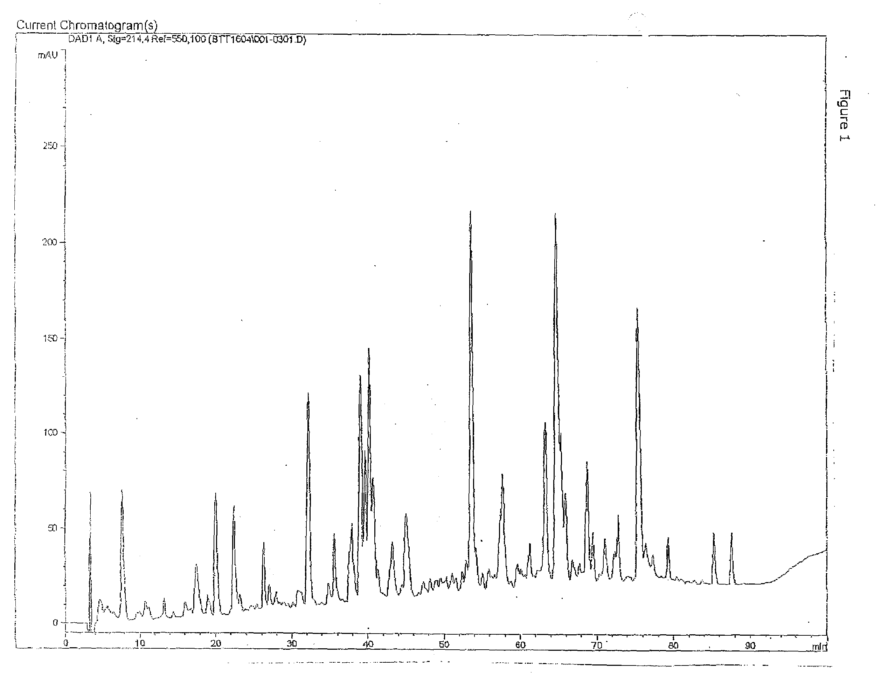

- Figure 1:: peptides (MW < or = 10 kDa) generated by chymotrypsin-cleavage of BLB

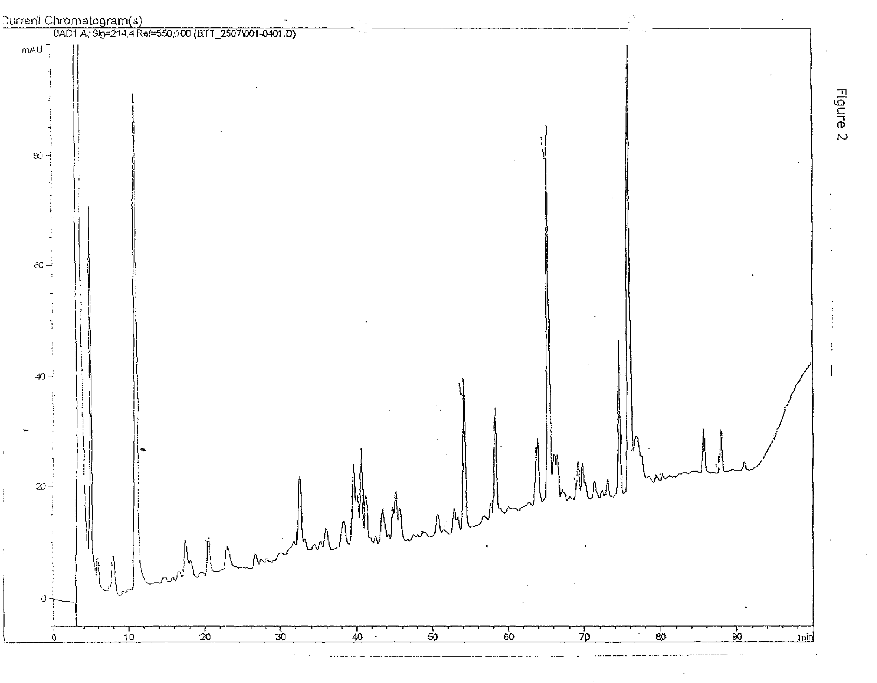

- Figure 2:: peptides from the chymotrypsin-cleaveage of BLG (MW < or = 10 kDa) that were bound to DnaK.

### DnaK.ATP preparation

25 µL of ATP solution (4.5 mg/mL) in buffer 1 (25 mM HEPES, 10 mM KCl, 3 mM MgCl₂, 5 mM 2-mercaptoethanol, pH 7.5) is added to 400 µL of DnaK (2 mg/mL of buffer 1). The solution is incubated at 20°C for one hour, and then is centrifuged through a centricon YM-10 assembly to remove any low molecular weight material loosely associated with Dna K. The large molecular weight fraction is removed, and washed extensively with buffer 1 by ultrafiltration using a centricon YM-10.

### In vitro production of the compositions

The ultrafiltrated digestion is diluted in the suitable buffer 1. Then, ADP is added (1 mM final) and the mixture is incubated for one hour at 25°C
or the ultrafiltrated digestion is mixed with the ADP-pretreated DnaK. Then, ADP is added (1 mM final) and the mixture is incubated for one hour at 25°C in the suitable buffer 1.

Both types of compositions are further diluted in the suitable buffer 1 to give the following compositions (total doses):
- p8:: 10 µg hydrolyzed BLG + 10 µg HSP
- p9:: 1 µg hydrolyzed BLG + 1 µg HSP
- p10:: 10 µg hydrolyzed BLG
- p11:: 1µg hydrolyzed BLG
- c:: control (buffer)

### Animal studies

Four groups of mice were sensitized against BLG at days J0, J7, J14 and J21 by gavage after gastric incubation with 20 mg BLG and 10 µg cholera toxin in 0.2 M Na₂HCO₃.

The compositions are administered in 5 equivalent doses (total dose divided by 5) every two days from the first day of the treatment (J26).

Mice are individually treated, and oral administration is performed by buccal injection in micro-does of 0.012 mL.

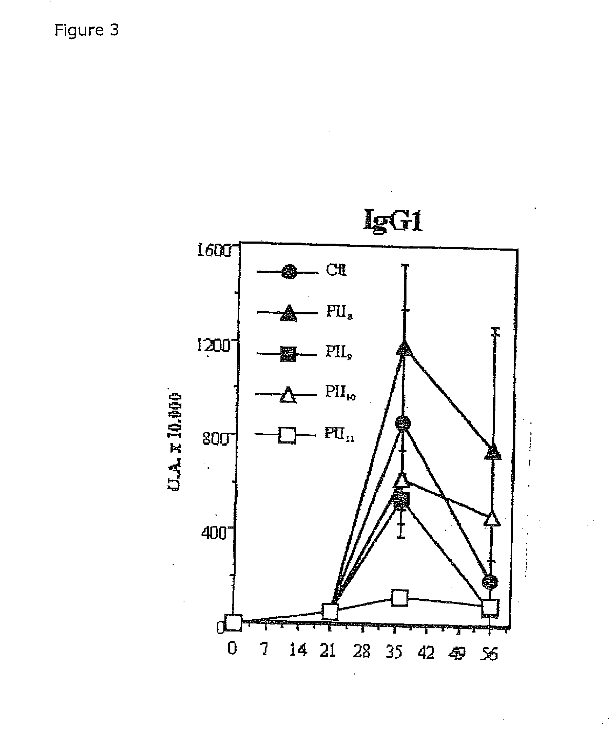

On day 36 and 56, immunglobulines were measured
- Figure 3: discloses the change of IgG1.

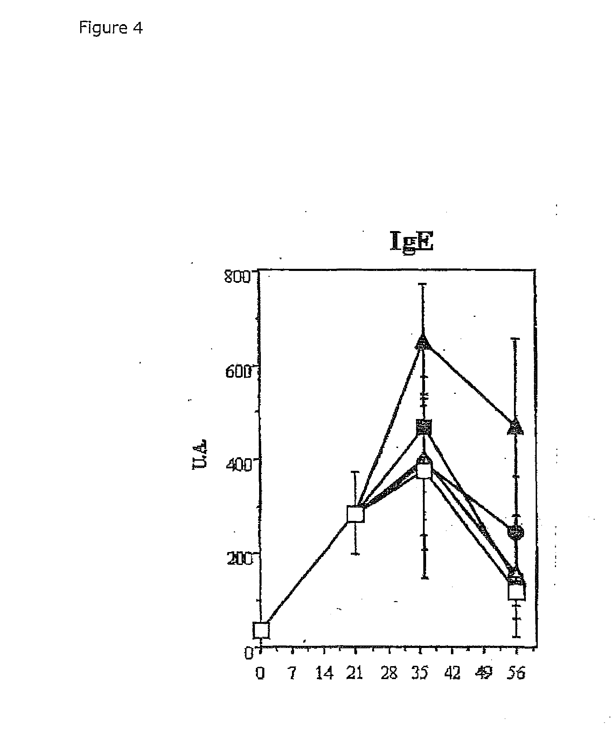

- Figure 4: discloses results for IgE.

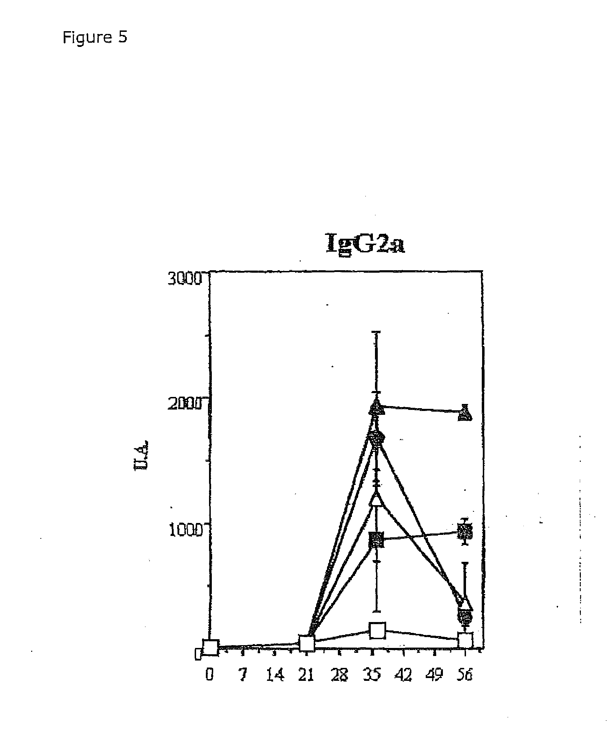

- Figure 5: discloses results for IgG2a.

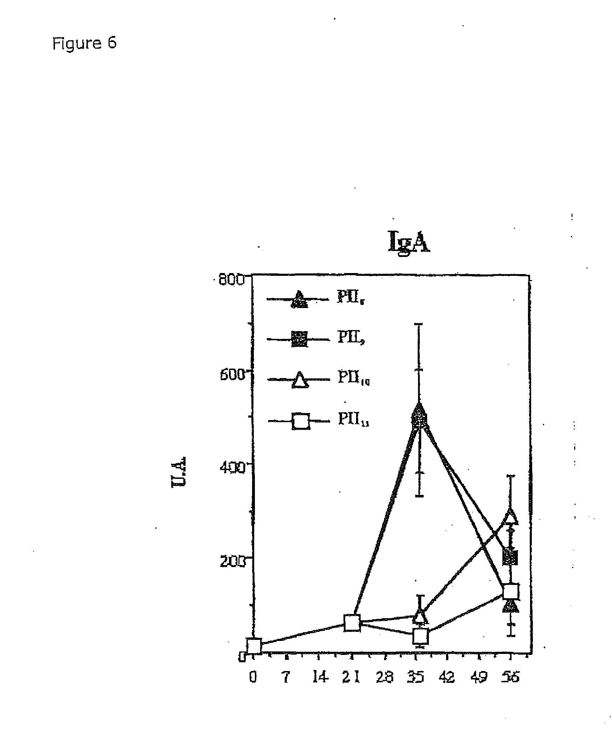

- Figure 6: discloses results for IgA.

It can be seen that the animals treated with peptides free of HSP show a reduced augmentation of immunglobulines. For IgE a composition comprising peptides alone is similar to the control group.

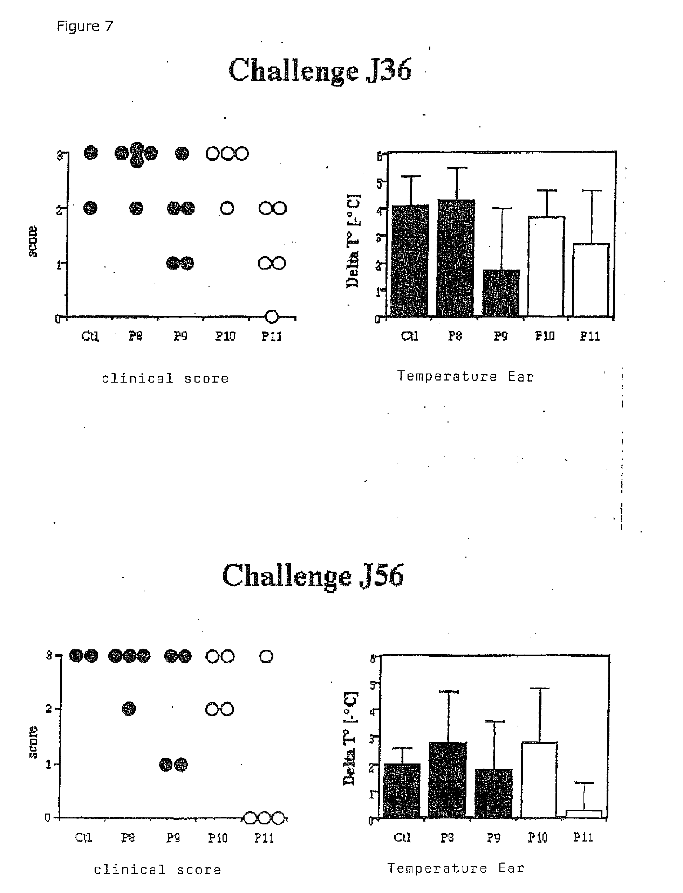

Figure 7 gives clinic scores for the different groups.

As can be seen from these data, some of the animals show a reduced clinical score when treated with small amount of a pharmaceutical composition of the present invention (1 µg; P 11) compared to a higher amount (10 µg; P10). This study also shows that significant oral tolerance was reached when the oral dose of peptides was lower than 10 µg. Low amounts of a pharmaceutical composition of the present invention seems to suppress the specific humoral response towards BLG (IgG1 and IgG2a) from days 36 to 56, whereas a pharmaceutical composition of the present invention combined with an adjuvant (HSP) incuduces an oral tolerance with stabilization of the IgG2a levels from days 36 to 56.

### Example 2

### Biological studies

It is a model wherein NOD (Not Obese Diabetic) mice are treated after the onset of the auto-immune disease.

After the onset of the first signs of diabetes, 500 normal Langherhans islets from young NOD mice are grafted under the renal capsule of diabetic animal. Glycosuria and glycemia are then monitored daily. Mice are considered diabetic when a glucosuria is detected and glycemia exceeds 12 mmole/L (2.16 g/L) during two consecutive days. The first day of hyperglycemia is considered as the start of relapse.

### Preparation of peptides

Insulin was either digested with trypsin or chymotrypsin (final ratio (W/W) protein/protease of 100: 1). The resulting solution is centrifuged through a centricon YM-10 assembly to remove the remaining protein and protease.

### Treatment of mice

All treatments were started on the first day after the onset of the disease, that is the day before transplantation. Mice were treated by sublingual injections, one dose each two days, to achieve the total doses:
Group 1: Peptides from trypsin digestion (1 µg)
Group 2: Peptides from chymotrypsin digestion (1 µg)
Group 3: Buffer.

### Clinical outcomes

In non-treated NOD mice, the average delay before a relapse occurs is about 11 to 12 days. Considering that a delay exceeding 14 days results from a therapeutic effect, one notices that, in the group treated with peptides from chymotrypsin-digested insulin, proportion of delays exceeding 14 days is 4/6 (66%). In the other group treated with either peptides from trypsin-digested insulin or buffer, the proportion is 2/6 (33%). Thus, there is a therapeutic effect of the peptides of insulin from a chymotrypsin digestion given orally.

## Claims

1. A pharmaceutical composition for sublingual or buccal administration comprising at least one substance obtainable by hydrolysis with chymotrypsin of an antigenic structure which induces graft rejection, allergic reaction or autoimmune disease.

2. The pharmaceutical composition of claim 1 wherein the amount of the at least one substance is in the range of 0,001 to 1000 µg, preferably 1 to 100 µg.

3. The pharmaceutical composition of claim 1 or 2 wherein the at least one substance is obtainable by hydrolysis of a protein.

4. The pharmaceutical composition of any one of claim 1 to 3 wherein the at least one substance is a peptide.

5. The pharmaceutical composition of claim 4 wherein the peptide has a molecular weight of less than 30 kDA, preferably less than 10 kDa.

6. The pharmaceutical composition of any one of claim 1 to 5 comprising additionally at least one substance selected from the group of nucleoside triphosphates, nucleoside diphosphates, nucleoside monophosphates, nucleic acids, peptide nucleic acids, nucleosides or analogs thereof, immunosuppressive cytokines, compounds inducing expression of immunoproteasomes, 1,25-dihydroxyvitamin D3 or analogs thereof, lipopolysaccharides, endotoxins, heat shock proteins, thioredoxin with either NADPH or NADP-thioredoxin reductase, dithiothreitol, adrenergic receptor agonists such as salbutanol, adrenergic receptor antagonists such as butoxamine, compounds that regulate the expression of the adhesion molecule ICAM-1, N-acetyl-L-cysteine, γ-L-glutamyl-L-cysteinyl-glycine (reduced L-glutathione), alpha-2-macroglobulins, inducers for Foxp3 gene expression, flavonoids, isoflavonoids, pterocarpanoids, stilbenes such as resveratrol, tachykinin receptor antagonists, chymase inhibitors, a muco-adhesive agent for attaching the particle to the intestinal mucosal lining such as a plant lectin, zinc, zinc salts, polysaccharides, vitamins and bacterial lysates.

7. The pharmaceutical composition of any one of claim 1 to 6 wherein the antigenic structure is selected from insulin, thyroglobulin, thyroid peroxidase, type II collagen, gliadin, GAD65, proteolipid protein, S-antigen, acetylcholin receptor, haptenized colonic proteins, interphotoreceptor retinoid binding protein, myelin basic protein, myelin oligodendrocyte glycoprotein, peripheral nerve P2, cytoplasmic TSH receptor, intrinsic factor, lens proteins, platelets, nucleoproteins such as histones, heat shock proteins, MHC I, MHC II, MHC-peptides complexes, milk allergens, venom allergens, egg allergens, weed allergens, grass allergens, tree allergens, shrub allergens, flower allergens, grain allergens, fungi allergens, fruit allergens, berry allergens, nut allergens, seed allergens, bean allergens fish allergens, shellfish allergens, meat allergens, spices allergens, insect allergens, mite allergens, animal allergens, animal dander allergens, allergens of Hevea brasiliensis, coagulation factors and blood group antigens.

8. Use of the pharmaceutical composition according to any one of claims 1 to 7 for the treatment or prevention of graft rejection, allergic reaction or autoimmune disease.

9. Use of a composition according to any one of claims 1 to 8 for elicting oral tolerance and/or the induction of cells that may produce immunosuppressive cytokines, more preferably TGF-beta and/or IL-4 and/or IL-10.

10. A process for the preparation of the pharmaceutical composition of any one of claims 1 to 7 comprising the steps of
• hydrolyzing with chymotrypsin an antigenic structure which induces graft rejection, allergic reaction or autoimmune disease to obtain at least one substance
• formulating the at least one substance for sublingual or buccal administration.

11. A composition comprising at least one substance obtainable by hydrolysis with chymotrypsin of an antigenic structure which induces graft rejection, allergic reaction or autoimmune disease.

12. The pharmaceutical composition of claims 1 or 2 in a sublingual formulation.

13. The pharmaceutical composition of claims 1 or 2 in a buccal formulation.
